# EUROPEAN PATENT SPECIFICATION

(11) **EP 1 292 348 B1**
(45) Date of publication and mention of the grant of the patent: **23.03.2005**
(21) Application number: 01949377.4
(22) Date of filing: 05.06.2001
(51) Int. Cl.: A61M 15/00

(54) **MEDICAMENT DISPENSER**
MEDIKAMENTENSPENDER
DISTRIBUTEUR DE MEDICAMENT

(30) Priority: 21.06.2000 GB 0015034
(43) Date of publication of application: 19.03.2003
(60) Divisional application: 04023768.7
(73) Proprietor: GLAXO GROUP LIMITED, Greenford, Middlesex UB6 0NN (GB)
(72) Inventor: DAVIES, Michael Birsha, Ware, Herts. SG12 ODP (GB); RAND, Paul Kenneth, Ware, Herts. SG12 ODP (GB)
(74) Representative: Rice, Jason Neale
(86) International application number: PCT/EP2001/006304
(87) International publication number: WO 2001/097886

(56) References cited:
- EP-A- 0 428 380
- WO-A-00/35522
- WO-A-97/20589
- US-A- 4 678 106
- US-A- 5 921 237

## Description

The present invention relates to a medicament dispenser for use in the administration of medicament in powder form to a patient.

The use of inhaler devices in the administration of medicaments, for example in bronchodilation therapy, is well known. Such devices generally comprise a body or housing within which a medicament container is located. Known inhaler devices include those in which the medicament container is suitable for containing a bulk reservoir of dry powder medicament. Such devices also typically comprise a mechanism for metering a dose from the reservoir and for transferring the dose to a delivery position where it is available for inhalation by the patient. These devices tend to be relatively size efficient in that they enable a large number of doses to be contained within a relatively device volume.

A mouthpiece (or nozzle) is typically provided to the inhaler device, wherein 'in use' the mouthpiece communicates with the powder delivery position to allow passage of the metered medicament dose to the mouthpiece and thence, to the patient. In a typical dispensing operation the body of the device is held by the patient and the mouthpiece (or nozzle) of the inhaler device is placed in the mouth (or nose) of the patient. The patient inhales, thereby causing transfer of the medicament dose from the delivery position to the interior of the body of the patient.

When not in use it is desirable, from a hygiene standpoint that the mouthpiece is provided with some kind of protective cover. The cover desirably acts both to prevent build-up of dirt on the mouthpiece and to prevent ingress of dirt into the body of the device through the mouthpiece, which might then be subject to inhalation by a patient. It is desirable, from a hygiene standpoint, that the mouthpiece does not come into contact with the hands of the user during the mouthpiece uncovering process.

Various sorts of mouthpiece cover have been proposed including detachable caps and lids. It is however, desirable that the cover is in some way attached or mounted to the device to minimise the risk that the cover is misplaced or lost.

It is desirable that the operation of a medicament dispenser be straightforward and non-complex and in particular, that the number of separate steps involved in preparing the device for use be minimised. This is especially relevant where the device is designed for use in the delivery of medicament in emergency or rescue situations (e.g. asthma attacks) where simplicity and ease of use is paramount.

The Applicants have therefore now devised a medicament dispenser having a dispensing outlet cover in which the actions of the transfer of a dose from to a delivery position and the opening of the dispensing outlet cover to reveal the dispensing outlet are operable in tandem. The device is therefore simple and straightforward to 'prime' for use by a patient.

WO96/08284 and WO98/30262 describe representative reservoir based dry powder medicament dispensers in which powder doses are metered from a reservoir to a delivery position in which the dose is inhalable by the patient. WO00/35522 describes a reservoir based dry powder medicament dispenser in which the metering system employs a rotatable auger to transfer powder from the reservoir to the delivery position. US-A-6,032,666 describes a medicament dispenser having a mouthpiece cover which is rotationally mounted to the body of the device and rotatable from a first position in which the mouthpiece is covered thereby to a second position in which the mouthpiece is exposed. WO 97/20589 describes a medicament dispenser according to the preamble of claim 1, in which dose is moved in response to pulling off of a protective cap from an outer casing.

According to one aspect of the present invention there is provided a medicament dispenser for dispensing medicament comprising a body having a dispensing outlet; within said body, a medicament container for containing medicament; a dose mover for moving a dose of medicament, said dose mover reversibly movable from an initial position to a dispensing position in which said dose of medicament is presented at the dispensing outlet; and a dispensing outlet cover, movable relative to the body from a storage position in which the dispensing outlet is covered to an in-use position in which the dispensing outlet is exposed, wherein rotational movement of the outlet cover from the storage position to the in-use position results in movement of the dose mover from the initial position to the dispensing position.

In one aspect, the medicament container is in the form of a reservoir container suitable for containing a reservoir of medicament, and said dose mover comprises a dose meter reversibly movable from an initial position wherein said dose meter communicates with the medicament container to receive a metered amount (e.g. volume or weight) of medicament therefrom to a dispensing position in which the dose meter communicates with the dispensing outlet.

Preferably, the dose meter comprises a metering recess therein. The metering recess may have any suitable shape such as an indent, a hemispherical cup, an elongate passage or a groove. It may meter a single dose of medicament or defined fraction or multiple thereof. Technical equivalents to the metering recess are envisaged including rod or shaft arrangements.

The medicament container may be provided with means to urge powder in the reservoir to a reservoir outlet that communicates in the initial position with the metering recess. The urging means may comprise various means of providing suitable biasing force such as spring loading means or vacuum means.

Suitably, one or more seals are provided between the dose meter and the medicament container. In one aspect, the seal comprises hard, incompressible mating surfaces which may be polished. In another aspect, the seal comprises a first mating surface formed from a hard, incompressible material and a second mating surface formed from a softer, more compressible material.

The seals may be formed by any suitable process including moulding processes such as one or two-shot moulding processes.

The seals may be comprised of any suitable sealing materials including organic polymeric materials, metals, resins or ceramics.

In another aspect, the medicament container is in the form of a medicament carrier suitable for carrying one or more discrete medicament doses, and the a dose mover comprises a mechanism for presenting at least one discrete medicament dose to a dispensing position in which the at least one discrete medicament dose communicates with the dispensing outlet.

Suitably, the carrier is in the form of a blister pack or pack having discrete doses of medicament printed or otherwise adhered thereto. Carriers for carrying a unit dose medicament are envisaged, as are those containing multiple unit doses arranged sequentially or otherwise, such as in series or matrix form. A particular multi-unit dose arrangement comprises an elongate strip having multiple unit doses of medicament arranged in series thereon (e.g. elongate blister strip).

The dose mover preferably incorporates or is coupled to a dose liberator for liberating the at least one medicament dose from the carrier. Where the at least one discrete medicament dose is comprised within a sealed pack (e.g. a blister pack) the dose liberator may for example, comprise a means for opening the pack by e.g. a rupture, piercing or separating action.

Once presented at the dispensing position, the at least one medicament dose may be protected by a local dose protector e.g. to prevent contamination thereof before delivery to the patient. The local dose protector may in one example, comprise a flap which covers the dose (thereby providing some protection thereto) during the time after presentation but before delivery to the patient. In another aspect, the dose protector comprises a mesh or cruciform shield to prevent access (e.g. finger access) to the presented dose.

Once the dose mover has been actuated and the dose moved to the dispensing position it desirable to be able to prevent movement of the dose back into the dispenser (e.g. feeding it back into a reservoir). The dose mover may therefore be provided with a mechanism (e.g. ratchet) which prevents such reverse movement until such a point as the dose has been delivered. Alternatively, the dose mover may be provided with a mechanism such that the dose mover is subject to an emptying action (e.g. transfer of remaining dose into a waste chamber) during the course of the reverse movement.

The movement of the dose mover and outlet cover may be arranged to be axial (i.e. in a straight line), arcuate, rotational or more complex. Preferably, the movement of the dose mover and outlet cover is of the same general type, that is to say e.g. both move axially, or in an arcuate sense or rotationally. More preferably, the movement is co-axial or co-arcuate or co-rotational sharing a common rotational axis.

In one aspect, the movement of the dose mover and outlet cover is coupled by a coupling mechanism. The coupling mechanism may comprise any suitable mechanism such as a gear mechanism, rack and pinion mechanism or hinged lever mechanism.

In another aspect, the movement of the dose mover and outlet cover is not directly coupled. Suitably, the dose mover and outlet cover share a common locus of movement, such a single axial track, a common pivot point or a common rotational axis.

The medicament container may be defined by the body. Alternatively, the medicament container is defined by an insert part. The insert may in one aspect comprise a cartridge which is reversibly locatable within the body. The body is typically arranged such that the cartridge is shaped for unique engagement therewith. Mechanical or electronic systems may be provided to enable recognition of the cartridge type including details of e.g. medicament type and recommended dose.

Suitably, the dose mover and/or outlet cover are provided with a safety trigger mechanism to prevent accidental movement thereof and/or false actuation. The mechanism may for example comprise a switch or latching mechanism.

Suitably, at least a portion of the outlet cover is shaped for ease of grip by the user.

Suitably, at least a portion of the outlet cover has a friction-enhancing coating for ease of grip by the user.

Suitably, the dispenser is provided with a mechanical or electronic dose counter, which indicates the number of doses dispensed from or remaining in the container. Preferably, the dose counter comprises an indexing mechanism actuated by movement of the dose mover and/or outlet cover relative to the body.

Suitably the outlet cover is actuable by a thumb motion. Preferably, the outlet cover is actuable by one-handed operation such as one in which the body is gripped within the cupped fingers of a user and the cover is moved by a thumb action. Alternatively, non-manual (e.g. electromechanical) mechanisms for opening the cover are envisaged.

The body, medicament container, seals or any other part of the device may comprise a desiccant material. The desiccant material may be applied as a coating to any surface or be impregnated within any part of the device or it may be contained within a suitable enclosure within the device. Suitable desiccants are selected from organic polymeric plastics, polyamides (e.g. nylon), silica gel, zeolites, alumina, bauxite, anhydrous calcium sulphate, activated bentonite day, water-absorbing clay, molecular sieves and any mixtures thereof.

Various electronic systems may be provided to the device including systems for monitoring actuation thereof; for counting the number of doses delivered or remaining; for checking compliance with a defined dose regime; for monitoring breath characteristics including peak flow; and for enabling unique recognition of a user such as by password protection. The electronic systems typically include some form of means for uploading data to the device from another computer system or downloading data from the device to another computer system.

According to a further aspect of the present invention there is provided a reloadable medicament dispenser for dispensing medicament comprising a body having a dispensing outlet; within said body, a loading position for reversible receipt of a cartridge comprising a medicament container for containing medicament; a dose mover reversibly movable from an initial position to a dispensing position in which the medicament is presented at the dispensing outlet; and a dispensing outlet cover, movable relative to the body from a storage position in which the dispensing outlet is covered to an in-use position in which the dispensing outlet is exposed, wherein the movement of the outlet cover from the storage position to the in-use position is coupled to the movement of the dose mover from the initial position to the dispensing position.

There is also provided a cartridge comprising a medicament container reversibly receivable by the reloadable medicament dispenser described above. The medicament container may be in reservoir form or in carrier form and the dose mover may have any of the forms as described hereinbefore. Typically, the cartridge will be reversibly retained within the medicament dispenser by some sort of latching or locking mechanism.

The reloadable medicament dispenser and cartridge therefor may be supplied separately or may be supplied as a kit of parts.

The cartridge itself is typically supplied charged with medicament. It may be arranged to be reloadable with medicament.

The medicament dispenser herein may be in the form of an inhaler device. Suitably, the dispensing outlet thereof defines a mouthpiece.

Herein the term 'mouthpiece' is used in a generic sense to mean an element shaped such as to be insertable into the mouth or nose of a patient for inhalation therethrough.

Suitably, the medicament dispenser herein comprises medicament. The medicament may in any suitable forms such as dry powder (either with excipient or excipient-free), liquid, solution, gaseous, suspension, tablet, capsule or nebule form. Preferably, the medicament is in dry powder form.

According to another aspect of the present invention, there is provided the use of a medicament dispenser provided herein for dispensing medicament.

Preferred embodiments of the medicament dispenser according to the present invention will now be described with reference to the accompanying drawings in which:
Fig. 1a is a schematic side view of a first inhalation device in accord with the present invention, wherein the mouthpiece is in the storage position;
Fig. 1b is a schematic side view of the device of Fig. 1a, wherein the mouthpiece is in the in-use position;
Fig. 2a is a sectional side view of a first medicament dispenser in accord with the present invention, wherein the mouthpiece is in the storage position; and
Fig. 2b is a sectional side view of the device of Fig. 2a, wherein the mouthpiece is in the in-use position.

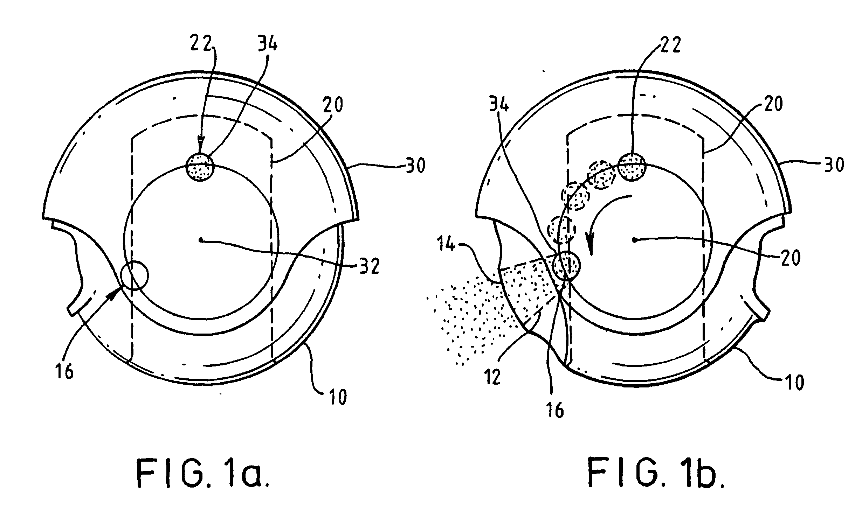

Figs. 1a and 1b show a first medicament dispenser herein in the open and closed positions. The device comprises a generally circular body 10 having an outlet 12 defining a mouthpiece 14. The body 10 is also provided with a dispensing orifice 16 which in the open position communicates with the outlet 12 and mouthpiece 14 for dispensing of medicament therethrough. Defined by the body 10 there is a medicament container 20 for containment of dry powder medicament. The container is provided with a delivery orifice 22 for delivery of powder. A cover 30 is provided to the body wherein the cover is pivotally mounted 32 to the body 10 such that it is rotatable by 180° around the body 10. The inner part of the cover 30 is provided with a metering recess 34. In the cover closed position, the metering recess 34 is located adjacent the delivery orifice 22 of the medicament container 20 such that powder may enter the metering recess 34 therefrom. In the open position, the metering recess 34 locates adjacent the dispensing orifice 16 such that powder may pass therethrough to the outlet 12 and mouthpiece 14.

It may be appreciated that the device is operable by a single-handed operation in which the cover 30 is held in the cupped fingers (not shown) of a user and the body 10 rotated through 180° by a thumb action of the user to bring the device from the open to closed position and vice versa. It may also be appreciated that the 180° rotation of the cover 30 acts such as to either expose or cover the mouthpiece 14 and move the metering recess 34 from a loading position to a dispensing position.

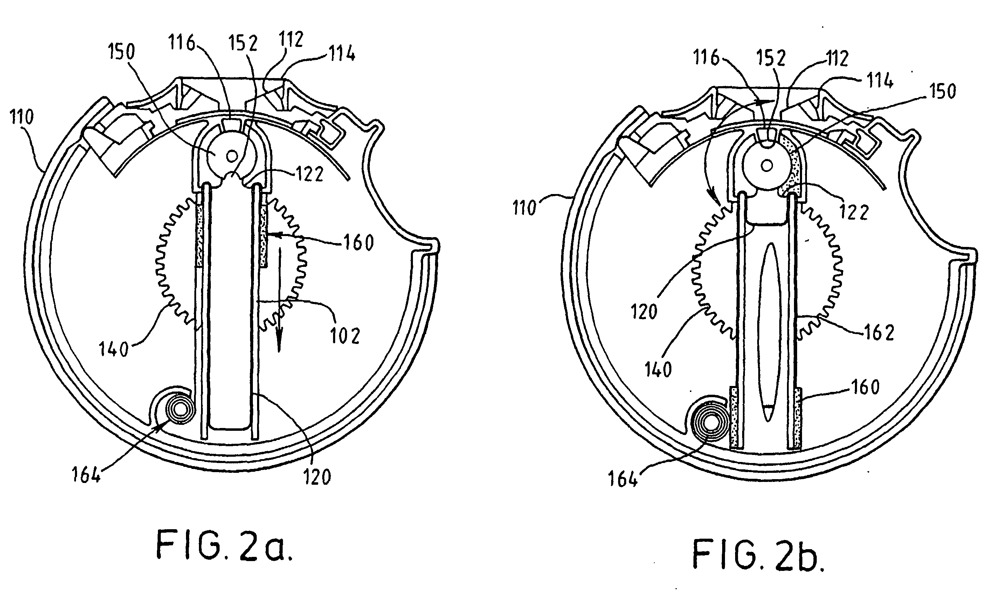

Figs. 2a and 2b show a second medicament dispenser herein in the open and closed positions. The device comprises a generally circular body 110 having an outlet 112 defining a mouthpiece 114. The body 110 is also provided with a dispensing orifice 116 which in the open position communicates with the outlet 112 and mouthpiece 114 for dispensing of medicament therethrough. Within the body 110 there is a collapsible tube container 120 for containment of dry powder medicament. The container 120 is provided with a delivery orifice 122 for delivery of powder. A cover (not visible) is provided to the body wherein the cover is pivotally mounted such that it is rotatable by 180° around the body 110. The cover is co-axially mounted and rotationally coupled to drive wheel 140 such that rotation of the cover results in rotation of the drive wheel 140. The drive wheel engages metering wheel 150 which is provided with a metering recess 152. In the cover-closed position, the metering recess 52 is located adjacent the delivery orifice 122 of the medicament cartridge 120 such that powder may enter the metering recess 152 therefrom. In the open position, the metering recess 152 locates adjacent the dispensing orifice 116 such that powder may pass therethrough to the outlet 112 and mouthpiece 114.

It may be seen that the container 120 is also provided with a system for ensuring constant delivery of powder to the delivery orifice 122. The system comprises a collar 160 movable along a track 162 located on either side of the tubular container 120. Pull is applied to the collar 160 by constant force spring 164. As the collar 160 is pulled along the track 162 by the action of the spring 164 the tube 120 is squeezed and powder is urged towards the delivery orifice 122.

It may be appreciated that the device is operable by a single-handed operation in which the cover (not shown) is held in the cupped fingers (not shown) of a user and the body 110 rotated through 180° by a thumb action of the user to bring the device from the open to closed position and vice versa. It may also be appreciated that the 180° rotation of the cover acts such as to both expose or cover the mouthpiece 114 and move the metering recess 152 from a loading position to a dispensing position.

The medicament dispenser herein is suitable for dispensing medicament, particularly for the treatment of respiratory disorders.

Appropriate medicaments may thus be selected from, for example, analgesics, e.g., codeine, dihydromorphine, ergotamine, fentanyl or morphine; anginal preparations, e.g., diltiazem; antiallergics, e.g., cromoglycate (eg s the sodium salt), ketotifen or nedocromil (eg as the sodium salt); antiinfectives e.g., cephalosporins, penicillins, streptomycin, sulphonamides, tetracyclines and pentamidine; antihistamines, e.g., methapyrilene; anti- inflammatories, e.g., beclomethasone (eg as the dipropionate ester), fluticasone (eg as the propionate ester), flunisolide, budesonide, rofleponide, mometasone eg as the furoate ester), ciclesonide, triamcinolone (eg as the acetonide) or 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester; antitussives, e.g., noscapine; bronchodilators, e.g., albuterol (eg as free base or sulphate), salmeterol (eg as xinafoate), ephedrine, adrenaline, fenoterol (eg as hydrobromide), formoterol (eg as fumarate), isoprenaline, metaproterenol, phenylephrine, phenylpropanolamine, pirbuterol (eg as acetate), reproterol (eg as hydrochloride), rimiterol, terbutaline (eg as sulphate), isoetharine, tulobuterol or 4-hydroxy-7-[2-[[2-[[3-(2-phenylethoxy)propyl]sulfonyl]ethyl]amino]ethyl-2(3H)-benzothiazolone; adenosine 2a agonists, eg 2R,3R,4S,5R)-2-[6-Amino-2-(1S-hydroxymethyl-2-phenyl-ethylamino)-purin-9-yl]-5-(2-ethyl-2H-tetrazol-5-yl)-tetrahydro-furan-3,4-diol (e.g. as maleate); α₄ integrin inhibitors eg (2S)-3-[4-({[4-(aminocarbonyl)-1-piperidinyl]carbonyl}oxy)phenyl]-2-[((2S)-4-methyl-2-{[2-(2-methylphenoxy) acetyl]amino}pentanoyl)amino] propanoic acid (e.g as free acid or potassium salt), diuretics, e.g., amiloride; anticholinergics, e.g., ipratropium (eg as bromide), tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagon; vaccines, diagnostics, and gene therapies. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament.

Preferred medicaments are selected from albuterol, salmeterol, fluticasone propionate and beclomethasone dipropionate and salts or solvates thereof, e.g., the sulphate of albuterol and the xinafoate of salmeterol.

Medicaments can also be delivered in combinations. Preferred formulations containing combinations of active ingredients contain salbutamol (e.g., as the free base or the sulphate salt) or salmeterol (e.g., as the xinafoate salt) or formoterol (eg as the fumarate salt) in combination with an anti-inflammatory steroid such as a bedomethasone ester (e.g., the dipropionate) or a fluticasone ester (e.g., the propionate) or budesonide. A particularly preferred combination is a combination of fluticasone propionate and salmeterol, or a salt thereof (particularly the xinafoate salt). A further combination of particular interest is budesonide and formoterol (e.g. as the fumarate salt).

It may be appreciated that any of the parts of the medicament container used therewith which contact the medicament may be coated with materials such as fluoropolymer materials which reduce the tendency of medicament to adhere thereto. Suitable fluoropolymers include polytetrafluoroethylene (PTFE) and fluoroethylene propylene (FEP). Any movable parts may also have coatings applied thereto which enhance their desired movement characteristics. Frictional coatings may therefore be applied to enhance frictional contact and lubricants used to reduce frictional contact as necessary.

It will be understood that the present disclosure is for the purpose of illustration only and the invention extends to modifications, variations and improvements thereto.

The application of which this description and claims form part may be used as a basis for priority in respect of any subsequent application. The claims of such subsequent application may be directed to any feature or combination of features described therein. They may take the form of product, method or use claims and may include, by way of example and without limitation, one or more of the following claims:

## Claims

1. A medicament dispenser for dispensing medicament comprising
a body (10; 110) having a dispensing outlet (12; 112);
within said body, a medicament container (20; 120) for containing medicament;
a dose mover (34; 150) for moving a dose of medicament, said dose mover reversibly movable from an initial position to a dispensing position in which said dose of medicament is presented at the dispensing outlet; and
a dispensing outlet cover (30), movable relative to the body from a storage position in which the dispensing outlet is covered to an in-use position in which the dispensing outlet is exposed,
**characterized in that** rotational movement of the outlet cover from the storage position to the in-use position results in movement of the dose mover from the initial position to the dispensing position,
with the proviso that the dose mover is not a rotatable auger.

2. A medicament dispenser according to claim 1, wherein said medicament container is suitable for containing a reservoir of medicament, and said dose mover comprises a dose meter reversibly movable from an initial position wherein said dose meter communicates with the medicament container to receive a metered amount of medicament therefrom to a dispensing position in which the dose meter communicates with the dispensing outlet.

3. A medicament dispenser according to claim 2, wherein the dose meter comprises a metering recess having a form selected from the group consisting of an indent, a hemispherical cup, an elongate passage and a groove.

4. A medicament dispenser according to either of claims 2 or 3, additionally comprising urging means (164) to urge medicament in the reservoir to a reservoir outlet of the medicament container that communicates in the initial position with the dose meter.

5. A medicament dispenser according any of claims 2 to 4, wherein one or more seals are provided between the dose meter and the medicament container.

6. A medicament dispenser according to claim 1, wherein said medicament container is a medicament carrier suitable for carrying one or more discrete medicament doses, and the dose mover comprises a mechanism for presenting at least one discrete medicament dose to a dispensing position in which the at least one discrete medicament dose communicates with the dispensing outlet.

7. A medicament dispenser according to claim 6, wherein the carrier is in the form of a blister pack.

8. A medicament dispenser according to either of claims 6 or 7, wherein the carrier is in the form of an elongate strip.

9. A medicament dispenser according to any of claims 6 to 8, wherein the dose mover incorporates or is coupled to a dose liberator for liberating the at least one medicament dose from the carrier.

10. A medicament dispenser according to any of claims 6 to 9, additionally comprising a dose protector to protect the at least one medicament dose at the dispensing position.

11. A medicament dispenser according to any of claims 1 to 10, additionally comprising a mechanism to prevent reverse movement of the dose mover prior to delivery of the dose.

12. A medicament dispenser according to any of claims 1 to 11, additionally comprising a mechanism to empty the dose mover during reverse movement thereof.

13. A medicament dispenser according to any of claims 1 to 12, wherein the dose mover and the outlet cover are movable in a common fashion.

14. A medicament dispenser according to any of claims 1 to 13, wherein the medicament container is defined by the body.

15. A medicament dispenser according to any of claims 1 to 13, wherein the medicament container is defined by an insert part.

16. A medicament dispenser according to claim 15, wherein the insert comprises a cartridge which is reversibly locatable within the body.

17. A medicament dispenser according to any of claims 1 to 16, wherein the dose mover and/or outlet cover are provided with a safety trigger mechanism to prevent accidental movement thereof.

18. A medicament dispenser according to any of claims 1 to 17, additionally comprising a mechanical or electronic dose counter.

19. A medicament dispenser according to any of claims 1 to 18, wherein the outlet cover is manually movable.

20. A medicament dispenser according to any of claims 1 to 19, additionally comprising a desiccant material located therein.

21. A medicament dispenser according to any of claims 1 to 20 in the form of an inhaler device, wherein the dispensing outlet defines a mouthpiece (14; 114).

22. A medicament dispenser according to any of claims 1 to 21, wherein the medicament container is charged with medicament.

23. A medicament dispenser according to claim 22, wherein the medicament is in a form selected from the group consisting of dry powder, liquid, solution, gaseous, suspension, tablet, capsule and nebule.

24. A medicament dispenser according to claim 23 additionally wherein the medicament is in dry powder form.

25. A medicament dispenser according to any of claims 22 to 24, wherein the medicament is suitable for the treatment of respiratory disorders.

26. A medicament dispenser according to any of claims 22 to 25, wherein the medicament is salmeterol xinafoate.

27. A medicament dispenser according to any of claims 22 to 25, wherein the medicament is fluticasone propionate.

28. A medicament dispenser according to any of claims 22 to 25, wherein the medicament is a combination of salmeterol xinafoate and fluticasone propionate.

29. A medicament dispenser according to any of claims 1 to 28, in the form of a cartridge reload, wherein the body defines a cartridge reload body for reversible receipt by a reloadable medicament dispenser.

30. A reloadable medicament dispenser according to claim 29 comprising an outer body; and reversibly received by said outer body, a medicament dispenser, in the form of a cartridge reload.

## Patentansprüche

1. Arzneimittelausgabevorrichtung zum Ausgeben eines Arzneimittels mit:
einem Korpus (10; 110), der eine Ausgabeöffnung (12; 112) besitzt;
einem Arzneimittelbehälter (20; 120) innerhalb des Korpus zum Aufnehmen des Arzneimittels;
einem Dosisbeweger (34; 150) zum Bewegen einer Arzneimitteldosis, der von einer ursprünglichen Position in eine Ausgabeposition, in der sich die Arzneimitteldosis an der Ausgabeöffnung befindet, umkehrbar bewegbar ist; und
einer Ausgabeöffnungsabdeckung (30), die relativ zu dem Korpus von einer Speicherposition, in der die Ausgabeöffnung bedeckt ist, in eine Benutzungsposition, in der die Ausgabeöffnung offen ist, bewegbar ist,
**dadurch gekennzeichnet, dass** eine Drehbewegung der Öffnungsabdeckung von der Speicherposition in die Benutzungsposition zu einer Bewegung des Dosisbewegers von der ursprünglichen Position in die Ausgabeposition führt, und zwar mit der Maßgabe, dass der Dosisbeweger keine drehbare Schnecke ist.

2. Arzneimittelausgabevorrichtung nach Anspruch 1, bei der der Arzneimittelbehälter zum Aufnehmen eines Arzneimittelreservoirs geeignet ist, und der Dosisbeweger eine Dosiereinheit aufweist, die von einer ursprünglichen Position, in der die Dosiereinheit in Verbindung mit dem Arzneimittelbehälter steht, um eine dosierte Menge an Arzneimittel aufzunehmen, in eine Ausgabeposition, in der die Dosiereinheit mit der Ausgabeöffnung in Verbindung steht, umkehrbar bewegbar ist.

3. Arzneimittelausgabevorrichtung nach Anspruch 2, bei der die Dosiereinheit eine Dosieraussparung aufweist, die eine Form besitzt, welche aus der Gruppe bestehend aus einer Vertiefung, einem halbkugelförmigen Becher, einer länglichen Durchführung und einer Nut ausgewählt ist.

4. Arzneimittelausgabevorrichtung nach Anspruch 2 oder 3, zusätzlich mit einem Treibmittel (164), um das Arzneimittel in dem Reservoir zu einer Reservoiröffnung, die in der ursprünglichen Position mit der Dosiereinheit in Verbindung steht, des Arzneimittelbehälters zu treiben.

5. Arzneimittelausgabevorrichtung nach einem der Ansprüche 2 bis 4, bei der eine oder mehrere Dichtungen zwischen der Dosiereinheit und dem Arzneimittelbehälter vorgesehen ist/sind.

6. Arzneimittelausgabevorrichtung nach Anspruch 1, bei der der Arzneimittelbehälter ein Arzneimittelträger ist, der zum Tragen von einer oder mehreren diskreten Arzneimitteldosen geeignet ist, und der Dosisbeweger einen Mechanismus zum Bewegen mindestens einer diskreten Arzneimitteldosis in eine Ausgabeposition, in der die mindestens eine diskrete Arzneimitteldosis mit der Ausgabeöffnung in Verbindung steht, aufweist.

7. Arzneimittelausgabevorrichtung nach Anspruch 6, bei der der Träger in Form einer Blisterpackung vorliegt.

8. Arzneimittelausgabevorrichtung nach Anspruch 6 oder 7, bei der der Träger in der Form eines länglichen Streifens vorliegt.

9. Arzneimittelausgabevorrichtung nach einem der Ansprüche 6 bis 8, bei der der Dosisbeweger einen Dosislöser enthält oder mit einem solchen gekoppelt ist zum Lösen der mindestens einen Arzneimitteldosis von dem Träger.

10. Arzneimittelausgabevorrichtung nach einem der Ansprüche 6 bis 9, zusätzlich mit einem Dosisprotektor zum Schützen der mindestens einen Arzneimitteldosis in der Ausgabeposition.

11. Arzneimittelausgabevorrichtung nach einem der Ansprüche 1 bis 10, zusätzlich mit einem Mechanismus, der eine Umkehrbewegung des Dosisbewegers vor dem Abgeben der Dosis verhindert.

12. Arzneimittelausgabevorrichtung nach einem der Ansprüche 1 bis 11, zusätzlich mit einem Mechanismus, der den Dosisbeweger während der Umkehrbewegung leert.

13. Arzneimittelausgabevorrichtung nach einem der Ansprüche 1 bis 12, bei der der Dosisbeweger und die Ausgabeöffnungsabdeckung gemeinsam bewegbar sind.

14. Arzneimittelausgabevorrichtung nach einem der Ansprüche 1 bis 13, bei der der Arzneimittelbehälter durch den Korpus bestimmt ist.

15. Arzneimittelausgabevorrichtung nach einem der Ansprüche 1 bis 13, bei der der Arzneimittelbehälter durch ein Einsatzteil bestimmt ist.

16. Arzneimittelausgabebehälter nach Anspruch 15, bei der der Einsatz ein Magazin aufweist, das in dem Korpus umkehrbar einbringbar ist.

17. Arzneimittelausgabevorrichtung nach einem der Ansprüche 1 bis 16, bei der der Dosisbeweger und/oder die Öffnungsabdeckung mit einem Sicherheitsauslösemechanismus versehen ist, um eine zufällige Bewegung derselben zu verhindern.

18. Arzneimittelausgabevorrichtung nach einem der Ansprüche 1 bis 17, zusätzlich mit einem mechanischen oder elektronischen Dosiszähler.

19. Arzneimittelausgabevorrichtung nach einem der Ansprüche 1 bis 18, bei der die Öffnungsabdeckung manuell bewegbar ist.

20. Arzneimittelausgabevorrichtung nach einem der Ansprüche 1 bis 19, zusätzlich mit einem darin befindlichen Trockenmittel.

21. Arzneimittelausgabevorrichtung nach einem der Ansprüche 1 bis 20 in der Form einer Inhaliervorrichtung, bei der die Ausgabeöffnung ein Mundstück (14; 114) bestimmt.

22. Arzneimittelausgabevorrichtung nach einem der Ansprüche 1 bis 21, bei der der Arzneimittelbehälter ein Arzneimittel enthält.

23. Arzneimittelausgabevorrichtung nach Anspruch 22, bei der das Arzneimittel in einer Form vorliegt, die ausgewählt ist aus der Gruppe: als Trockenpulver, flüssig, als Lösung, gasförmig, als Suspension, als Tablette, als Kapsel und als Nebel.

24. Arzneimittelausgabevorrichtung nach Anspruch 23, bei der das Arzneimittel in Trockenpulverform vorliegt.

25. Arzneimittelausgabevorrichtung nach einem der Ansprüche 22 bis 24, bei der das Arzneimittel für die Behandlung von Atemwegserkrankungen geeignet ist.

26. Arzneimittelausgabevorrichtung nach einem der Ansprüche 22 bis 25, bei der das Arzneimittel gleich Salmeterolxinafoat ist.

27. Arzneimittelausgabevorrichtung nach einem der Ansprüche 22 bis 25, bei der das Arzneimittel gleich Fluticasonpropionat ist.

28. Arzneimittelausgabevorrichtung nach einem der Ansprüche 22 bis 25, bei der das Arzneimittel eine Kombination aus Salmeterolxinafoat und Fluticasonpropionat ist.

29. Arzneimittelausgabevorrichtung nach einem der Ansprüche 1 bis 28, vorliegend in der Form einer Magazinladung, bei der der Korpus einen Magazinladekorpus bestimmt zur umkehrbaren Aufnahme von einer wiederauffüllbaren Arzneimittelausgabevorrichtung.

30. Wiederauffüllbare Arzneimittelausgabevorrichtung nach Anspruch 29 mit einem äußeren Korpus und einer Arzneimittelausgabevorrichtung in der Form einer Magazinladung, die von dem äußeren Korpus umkehrbar aufgenommen werden kann.

## Revendications

1. Distributeur de médicament pour distribuer un médicament comprenant
un corps (10 ; 110) ayant une sortie de distribution (12 ; 112) ;
à l'intérieur dudit corps, un récipient à médicament (20 ; 120) pour contenir un médicament ;
un dispositif de déplacement de dose (34 ; 150) pour déplacer une dose de médicament, ledit dispositif de déplacement de dose étant mobile de façon réversible à partir d'une position initiale vers une position de distribution dans laquelle ladite dose de médicament est présentée au niveau de la sortie de distribution ; et
un couvercle de sortie de distribution (30), mobile par rapport au corps à partir d'une position de rangement dans laquelle la sortie de distribution est recouverte jusqu'à une position d'utilisation dans laquelle la sortie de distribution est exposée,
**caractérisé en ce que** le mouvement de rotation du couvercle de sortie de la position de rangement vers la position d'utilisation entraîne le mouvement du dispositif de déplacement de dose de la position initiale vers la position de distribution,
avec la condition que le dispositif de déplacement de dose n'est pas un tourniquet rotatif.

2. Distributeur de médicament selon la revendication 1, dans lequel ledit récipient à médicament est adapté pour contenir un réservoir de médicament, et ledit dispositif de déplacement de dose comprend un appareil de mesure de dose mobile de façon réversible à partir d'une position initiale dans laquelle ledit appareil de mesure de dose communique avec le récipient à médicament pour recevoir une quantité mesurée de médicament à partir de celui-ci jusqu'à une position de distribution dans laquelle l'appareil de mesure de dose communique avec la sortie de distribution.

3. Distributeur de médicament selon la revendication 2, dans lequel l'appareil de mesure de dose comprend un évidement de dosage ayant une forme sélectionnée parmi le groupe constitué d'une entaille, une coquille hémisphérique, un passage allongé et une rainure.

4. Distributeur de médicament selon l'une quelconque des revendications 2 ou 3, comprenant en outre des moyens de poussée (164) pour pousser un médicament dans le réservoir jusqu'à une sortie de réservoir du récipient à médicament qui communique dans la position initiale avec l'appareil de mesure de dose.

5. Distributeur de médicament selon l'une quelconque des revendications 2 à 4, dans lequel un ou plusieurs joints sont prévus entre l'appareil de mesure de dose et le récipient à médicament.

6. Distributeur de médicament selon la revendication 1, dans lequel ledit récipient à médicament est un dispositif porteur de médicament approprié pour porter une ou plusieurs doses de médicament distinctes, et le dispositif de déplacement de dose comprend un mécanisme pour présenter au moins une dose de médicament distincte à une position de distribution dans laquelle la au moins une dose de médicament distincte communique avec la sortie de distribution.

7. Distributeur de médicament selon la revendication 6, dans lequel le dispositif porteur a la forme d'un emballage-coque.

8. Distributeur de médicament selon l'une quelconque des revendications 6 ou 7, dans lequel le dispositif porteur est la forme d'une bande allongée.

9. Distributeur de médicament selon l'une quelconque des revendications 6 à 8, dans lequel le dispositif de déplacement de dose incorpore, ou est couplé à, un libérateur de dose pour libérer la au moins une dose de médicament à partir du dispositif porteur.

10. Distributeur de médicament selon l'une quelconque des revendications 6 à 9, comprenant en outre un protecteur de dose pour protéger la au moins une dose de médicament au niveau de la position de distribution.

11. Distributeur de médicament selon l'une quelconque des revendications 1 à 10, comprenant en outre un mécanisme pour empêcher le mouvement de retour du dispositif de déplacement de dose avant la distribution de la dose.

12. Distributeur de médicament selon l'une quelconque des revendications 1 à 11, comprenant en outre un mécanisme pour vider le dispositif de déplacement de dose au cours du mouvement de retour de celui-ci.

13. Distributeur de médicament selon l'une quelconque des revendications 1 à 12, dans lequel le dispositif de déplacement de dose et le couvercle de sortie sont mobiles de façon commune.

14. Distributeur de médicament selon l'une quelconque des revendications 1 à 13, dans lequel le récipient à médicament est défini par le corps.

15. Distributeur de médicament selon l'une quelconque des revendications 1 à 13, dans lequel le récipient à médicament est défini par une partie rapportée.

16. Distributeur de médicament selon la revendication 15, dans lequel la partie rapportée comprend une cartouche qui est positionnable de façon réversible à l'intérieur du corps.

17. Distributeur de médicament selon l'une quelconque des revendications 1 à 16, dans lequel le dispositif de déplacement de dose et/ou couvercle de sortie est/sont pourvu(s) d'un mécanisme d'enclenchement de sécurité pour empêcher le mouvement accidentel de celui/ceux-ci.

18. Distributeur de médicament selon l'une quelconque des revendications 1 à 17, comprenant en outre un compteur de dose mécanique ou électronique.

19. Distributeur de médicament selon l'une quelconque des revendications 1 à 18, dans lequel le couvercle de sortie est mobile manuellement.

20. Distributeur de médicament selon l'une quelconque des revendications 1 à 19, comprenant en outre un matériau dessiccatif situé dans celui-ci.

21. Distributeur de médicament selon l'une quelconque des revendications 1 à 20, sous forme d'un dispositif d'inhalation, dans lequel la sortie de distribution définit un embout buccal (14 ; 114).

22. Distributeur de médicament selon l'une quelconque des revendications 1 à 21, dans lequel le récipient à médicament est rempli avec le médicament.

23. Distributeur de médicament selon la revendication 22, dans lequel le médicament est de forme sélectionnée parmi le groupe constitué de poudre sèche, liquide, solution, gaz, suspension, tablette, capsule et produit nébulisé.

24. Distributeur de médicament selon la revendication 23, dans lequel en outre le médicament est sous forme de poudre sèche.

25. Distributeur de médicament selon l'une quelconque des revendications 22 à 24, dans lequel le médicament est adapté pour le traitement de maladies respiratoires.

26. Distributeur de médicament selon l'une quelconque des revendications 22 à 25, dans lequel le médicament est du xinafoate de salmétérol.

27. Distributeur de médicament selon l'une quelconque des revendications 22 à 25, dans lequel le médicament est du propionate de fluticasone.

28. Distributeur de médicament selon l'une quelconque des revendications 22 à 25, dans lequel le médicament est une combinaison de xinafoate de salmétérol et propionate de fluticasone.

29. Distributeur de médicament selon l'une quelconque des revendications 1 à 28, sous forme d'une recharge de cartouche, dans lequel le corps définit un corps de recharge de cartouche pour une réception réversible par un distributeur de médicament rechargeable.

30. Distributeur de médicament rechargeable selon la revendication 29, comprenant un corps extérieur ; et reçu de façon réversible par ledit corps extérieur, un distributeur de médicament, sous forme d'une recharge de cartouche.
